Europäisches Patentamt

European Patent Office

Office européen des brevets

(19)

(11) Publication number : **0 437 367 A2**

(12) # EUROPEAN PATENT APPLICATION

(21) Application number : 91300179.8

(22) Date of filing : 10.01.91

(51) Int. Cl.⁵ : **C07K 7/08,** C07K 7/10, A61K 37/02

The applicant has filed a statement in accordance with Rule 28 (4) EPC (issue of a sample only to an expert). Accession number(s) of the deposit(s): ATCC 67873.

(30) Priority : 11.01.90 US 463685

(43) Date of publication of application :
17.07.91 Bulletin 91/29

(84) Designated Contracting States :
CH DE FR GB IT LI NL

(71) Applicant : MERCK & CO. INC.
126, East Lincoln Avenue P.O. Box 2000
Rahway New Jersey 07065-0900 (US)

(72) Inventor : Sato, Masahiko
10 Windsor Court
Lansdale, PA 19446 (US)
Inventor : Grasser, William A.
82 Carousel Circle
New Britain, PA 18901 (US)
Inventor : Gould, Robert J.
973 Gravel Pike
Greenland, PA 18054 (US)

(74) Representative : Hesketh, Alan, Dr. et al
European Patent Department Merck & Co.,
Inc. Terlings Park Eastwick Road
Harlow Essex, CM20 2QR (GB)

(54) Compositions and methods for inhibiting osteoclast cellular adhesion to bone.

(57) A composition and method for inhibiting osteoclast cellular adhesion to bone comprising a polypeptide of formula

X-Cys-R-R-R-Arg-Gly-Asp-R-R-R-R-R-Cys-Y

EP 0 437 367 A2

## BONE RESORPTION INHIBITOR

FIELD OF THE INVENTION

The invention relates to polypeptides for inhibiting attachment of osteoclast cells to bone.

BACKGROUND OF THE INVENTION

This invention relates generally to modulating cell adhesion, including inhibiting the binding of fibrinogen and other proteins to blood platelets, inhibiting the aggregation of blood platelets, and inhibiting osteoclast cellular adhesion to bone.

Fibrinogen is a glycoprotein, present in blood plasma, which participates in platelet aggregation and in fibrin formation. Platelets are cell-like anucleated fragments, found in the blood of all mammals, which participate in blood coagulation. Interaction of fibrinogen with a receptor, the platelet membrane glycoprotein complex IIb/IIIa, is known to be essential for normal platelet function.

When a blood vessel is damaged, platelets adhere to the disrupted subendothelial surface. The adherent platelets subsequently release biologically active constituents and aggregate. Aggregation is initiated by the binding of agonists, such as thrombin, epinephrine, or ADP to specific platelet membrane receptors. Stimulation by agonists results in exposure of latent fibrinogen receptors on the platelet surface, and binding of fibrinogen to the glycoprotein IIb/IIIa complex.

Attempts have been made to use natural products and synthetic peptides to study the mechanism of platelet aggregation and adhesion. Platelet aggregation inhibitor proteins have been isolated from a variety of viper venoms. However, studies of the mechanism of inhibition of platelet aggregation by these proteins have been hampered by the lack of structural information.

Recently, a venom peptide called trigramin was characterized in some detail (see Huang et al., J. of Biol. Chem., 1987, 262, pp. 16157-16163 and U.S. Serial No. 121,972, filed November 18, 1987). The peptide was reported to competitively inhibit fibrinogen binding to the glycoprotein IIb/IIIa complex on the platelet membrane. The peptide contains an Arg-Gly-Asp sequence which is believed to be close to the COOH-terminal residue.

Rouslahti and Pierschbacher, Science, 1987, 238, pp. 491-497, describe adhesive proteins such as fibronectin, vitronectin, osteopontin, collagens, thrombospondin, fibrinogen, and von Willebrand factor present in extracellular matrices and in the blood. The proteins contain the tripeptide arginine-glycine-aspartic acid as their cell recognition site. The tripeptides are recognized by at least one member of a family of structurally related receptors, integrins, which are heterodimeric proteins with two membrane-spanning subunits. The authors state that the conformation of the tripeptide sequence in the individual proteins may be critical to recognition specificity.

Cheresh, Proc. Nat'l. Acad. Sci. USA, 1987, 84, pp. 6471-6475, describes an Arg-Gly-Asp directed adhesion receptor expressed by human endothelial cells that is structurally similar to the IIb/IIIa complex on platelets but antigenically and functionally distinct. The receptor is directly involved in endothelial cell attachment to fibrinogen, von Willebrand factor, and vitronectin.

Pierschbacher and Rouslahti, J. of Biol. Chem, 1987, 262, 36, pp. 17294-17298 describe influence of stereochemistry of the sequence Arg-Gly-Asp-Xaa on binding specificity of peptides containing the tripeptide sequence Arg-Gly-Asp. In Proc. Nat'l. Acad. Sci. USA, 1984, 81, pp. 5985-5988, the same authors describe variants of the cell recognition site of fibronectin that retain attachment-promoting activity. The tetrapeptide Arg-Gly-Asp-Ser is described as the minimal structure recognized by cells in the large, adhesive glycoprotein fibronectin. Peptides having portions -Arg-Gly-Asp-Ser- are described in U.S. Patent Nos. 4,589,881 and 4,614,517. Peptides having portions -Arg-Gly-Asp-R wherein R is selected from Thr or Cys or other amino acid having the same cell-attachment activity as fibronectin, are described in U.S. Patent No. 4,578,079.

Ruggeri et al., Proc. Nat'l. Acad. Sci. USA, 1986, 83, pp. 5708-5712, describes a series of synthetic peptides, designed in lengths to 16 residues, that contain the sequence Arg-Gly-Asp-Val, which inhibit fibrinogen binding to platelets.

Gold et al., Circulation, 77, 3, March 1988, pp. 670-677 describes the effects of bolus injections of recombinant single-chain tissue-type plasminogen activator and of $F(ab')_2$ fragments of a murine monoclonal antibody (7E3) against the human platelet GPIIb/IIIa receptor on coronary arterial thrombolysis and reocclusion. Gold et al. found that injection of $F(ab')_2$ fragments of the monoclonal antibody 7E3, directed against the platelet GPIIb/IIIa receptor, accelerates thrombolysis with recombinant single-chain tissue-type plasminogen activator and prevents reocclusion.

While it is known that the tripeptide sequence Arg-Gly-Asp is present in certain polypeptides which can

EP 0 437 367 A2

duplicate or inhibit the cell attachment-promoting effects of fibronectin and vitronectin, there is little understanding of the influence on binding specificity of other amino acids in the polypeptide. Applicants have identified and purified polypeptides which contain the tripeptide sequence Arg-Gly-Asp, which are platelet aggregation inhibitors and which have cysteine residues specifically positioned in relation to the position of the tripeptide. The specific location of the cysteine amino acids appears to significantly influence the ability of these polypeptides to inhibit platelet aggregation.

The advent of reliable methods and equipment for the chemical synthesis of long oligodeoxynucleotides has made gene synthesis a feasible alternative to the isolation and expression of natural genes (Caruthers, Science, 230 (1985) pp. 281-285 ; Ferretti et al., Proc. Nat'1. Acad. Sci. USA, 83 (1986), pp. 599-603 ; Wosnick et al., Gene, 60 (1987) pp. 115-127 ; Nassal, Gene, 66 (1988) pp. 279-294 ; Bell et al., Gene, 63 (1988) pp. 155-161). The technique is especially useful for expressing and engineering small natural proteins. However, many eukaryotic proteins expressed in a bacterial host are not stable (Taniguchi et al. Proc. Nat'1. Acad. Sci. USA, 77 (1980) pp 5230-5233 ; Davis et al., Proc. Nat'1. Acad. Sci. USA, 78 (1981) pp. 5376-5380 ; Shen, Proc. Nat'1. Acad. Sci. USA, 81 (1984) pp. 4627-4631 ; and Lennick et al., Gene, 61 (1987) pp. 103-112). A frequent strategy used to circumvent the instability is to fuse the gene of interest to a nucleotide sequence coding for a bacterial protein to produce a fused protein, thereby protecting the eukaryotic protein. Many chemical reagents and proteases have been reported to cleave a variety of amino acid sites on fusion proteins. Cyanogen bromide efficiently and selectively cleaves at methionine (Savige et al., Methods in Enzymology, Hirs and Timashelff eds. Academic Press, New York, 47 (1977) pp. 459-469 ; Nagai et al., Nature, 309 (1984) pp. 810-812 ; Szoka et al., DNA, 5 (1986) pp. 11-20 ; and Haffey et al., DNA, 6 (1987) pp. 565-571).

Osteoclasts resorb mineralized tissue in vertebrates. Bone resorption appears to proceed by the intricate coordination of the processes of adhesion to bone, polarized secretion of acid and proteases, and active motility of osteoclasts along the bone substrate (Kanehisa and Heersche, Bone 9 : 73-79 (1988), Baron et al., J. Cell Biol. 106 : 1863-1872 (1988), Blair et al., Science 245 : 855-857 (1989), Zambonin Zallone et al., Exp. Cell Res. 182 : 645-652 (1989)). Cells active in resorption are tightly apposed to the bone surface and form specialized structures at this interface consisting of a highly convoluted membrane called the "ruffled border" surrounded by an actin rich region called the "clear zone" (Vases, Clin. Orthop. Relat. Res. 228 : 239-271 (1988), Sato and Rodan, "Cell Shape : Determinants, Regulation and Regulatory Role" Academic Press Inc., Orlando, FL pp. 329-362 (1989).

Integrins are heterodimeric glycoproteins which recogize the Arg-Gly-Asp (RGD) sequence and participate in both cell- substrate and cell- cell interactions (Hynes, Cell 48 : 549-554 (1987)). The integrin superfamily is subdivided into at least 4 families, defined by the highly disulfide- linked beta subunit. These are the VLA/ fibronectin (beta 1) receptors, the leukocyte Leu- CAM/CD18 (beta 2) receptors, the beta 3 receptors, and the epithelial cell beta 4 receptor (Kajiji et al., EMBO J. 8 : 673-680 (1989)). The beta 3 family, also called cytoadhesins, includes the platelet GP IIbIIIa complex that is essential for platelet aggregation and the vitronectin receptor which was immunologically detected on osteoclasts, osteoblasts, osteosarcomas and other cells (Horton et al., Cancer Res., 45 : 5663-5669 (1985), Dedhar et al. J. Cell Biol. 105 : 1175-1182 (1987), Zambonin Zallone et al. (1989)). In osteoclasts, the beta-3 integrins localize to bands of podosomes which may correspond to the clear zone of resorbing osteoclasts (Zambonin Zallone et al. (1989)).

SUMMARY OF THE INVENTION

The present invention comprises fibrinogen-receptor antagonist polypeptides characterized in that they inhibit fibrinogen-platelet binding and have the general formula I :

$$X-Cys-R-R-R-Arg-Gly-Asp-R-R-R-R-R-Cys-Y \quad (I)$$

wherein X is H or at least one amino acid ; Y is OH or at least one amino acid ; and each R, either the same or different, is any amino acid.

Polypeptides within the formula I include those of formula Ia :

$$H_2N-(Ch)-Cys-R-R-R-Arg-Gly-Asp-R-R-R-R-R-Cys-$$
$$(Cx)-H \quad (Ia)$$

wherein Ch represents at least one amino acid ; Cx represents at least one amino acid ; and each R-, either the same or different, represents an amino acid.

3

Polypeptides of the invention have been purified from the venom of various vipers, e.g. Trimeresurus gramineus, Echis carinatus, Agkistrodon piscivorus, Bitis arietans and Eristocophis macmahonii. These polypeptides are useful for inhibiting fibrinogen binding to human platelets and inhibiting fibrinogen-induced aggregation of human platelets.

These polypeptides are purified from viper venom according to the purification procedure described below. They are rich in cysteine and contain the common sequence -Cys-R-R-R-Arg-Gly-Asp-R-R-R-R-R-Cys-, where each R, either the same or different, represents any amino acid.

Although Applicants do not wish to be bound to any particular theory regarding binding mechanism, it is believed that the location of the cysteine amino acids plays an important role in determining the three-dimensional conformation, degree of rigidity and binding specificity of the polypeptides.

The present invention also comprises a gene or degenerate equivalent encoding a platelet aggregation inhibitor of the general formula :

$$X-Cys-R-R-R-Arg-Gly-Asp-R-R-R-R-R-Cys-Y$$

wherein X is H or at least one amino acid ; Y is OH or at least one amino acid ; and each R, either same or different, is any amino acid.

A preferred gene of the present invention encodes modified echistatin. Echistatin is defined as :

$$Glu-Cys-Glu-Ser-Gly-Pro-Cys-Cys-Arg-Asn-Cys-Lys-$$
$$Phe-Leu-Lys-Glu-Gly-Thr-Ile-Cys-Lys-Arg-Ala-Arg-$$
$$Gly-Asp-Asp-Met-Asp-Asp-Tyr-Cys-Asn-Gly-Lys-Thr-$$
$$Cys-Asp-Cys-Pro-Arg-Asn-Pro-His-Lys-Gly-Pro-Ala-$$
$$Thr$$

The menthionine at position 28 of the native protein is replaced by leucine in recombinant echistatin. The synthetic gene is expressed in E. coli (MB-5385, ATCC Accession No. 67873, deposited with American Type Culture Collection, 12301 Parklawn drive, Rockville, MD 20852 U.S.A.) using an expression vector pJC264, which was constructed by inserting portions of the E. coli cheB and cheY gene complex into the plasmid pUC13 (U.S. Serial No. 145,800, corresponding to EP 324647. Microorganisms of the strain have been deposited under the Budapest Treaty. High level expression of the r-leu 28-echistatin gene was achieved by fusion with the E. coli cheY gene in the expression vector. Recombinant-leu 28-echistatin was liberated from the fusion protein by cyanogen bromide cleavage at the methionine genetically inserted between the cheY protein and echistatin,and purified to homogeneity by reverse phase HPLC, and refolded. The refolded r-leu 28-echistatin is indistinguishable from native echistatin in inhibiting platelet aggregation, suggesting that the methionine at position 28 is not essential for the biological activity of this platelet aggregation inhibitor.

The invention also includes compositions for inhibiting platelet aggregation using proteins of the invention and expression vectors useful for production of recombinant proteins of the invention.

The invention also includes synthetic. polypeptides of formula I and methods of synthesis.

The invention also includes compositions and methods for inhibiting osteoclast cellular adhesion to bone by administering to the bone an effective amount of a polypeptide of the invention.

## DETAILED DESCRIPTION OF THE INVENTION

Native proteins as used herein refers to full length proteins produced by the corresponding genes. Recombinant proteins refers to the isolation of a gene or cDNA for a desired protein and the use of that purified gene or cDNA to construct a cell which will overproduce the desired protein. Microheterogeneous forms as used herein refers to a single gene product, that is a protein produced from a single gene unit of DNA, which is structurally modified following translation. These structural modifications, however, do not result in any significant alterations of the activity of the protein. The modifications may take place either in vivo or during the isolation and purification process. In vivo modification may result in, but is not limited to, acetylation at the N-terminus, proteolysis, glycosylation or phosphorylation. Proteolysis may include exoproteolysis wherein one or more terminal amino acids are sequentially, enzymatically cleaved to produce microheterogeneous forms which have fewer amino acids than the original gene product. Proteolysis may also include endoproteolytic modification that results from the action of endoproteases which cleave the peptide at specific locations within the amino acid sequence. Similar modifications can occur during the purification process which may result in the production of microheterogeneous forms. The most common modification occuring during purification is proteolysis.

Recombinant DNA technology is defined herein as technology which allows segments of genetic information, DNA, from different cells, usually from different organisms, to be joined end-to-end outside the organisms from which the DNA was obtained and to incorporate this hybrid DNA into a cell that will allow the production of the protein for which the original DNA encodes. Genetic information, DNA or mRNA, is isolated and incorporated into an appropriate cloning vector, and transduced into an appropriate host cell.

Cloning vector as used herein is defined as a DNA sequence which allows the incorporation of specific experimental foreign DNA, with the combined DNA being introduced into a host cell that can exist in a stable manner and express the protein dictated by the experimental DNA. The foreign DNA combined with the vector DNA constitutes a recombinant DNA molecule which is derived from recombinant technology. Cloning vectors may include plasmids, bacteriophage, viruses and cosmids. Expression vectors are defined herein as DNA sequences that are required for the transcription of cloned copies of genes and the translation of their mRNAs in an appropriate host. Such vectors can be used to express either procaryotic or eucaryotic genes in a variety of cells such as bacteria, yeast, insect and mammalian cells. The proteins may also be expressed in a number of virus systems. An appropriately constructed expression vector should contain : an origin of replication for autonomous replication in host cells, selective markers, a limited number of useful restriction enzyme sites, a high copy number, and strong promoters. A promoter is defined as a DNA sequence that directs RNA polymerase to bind to DNA and to initiate RNA synthesis. A strong promoter is one which causes mRNAs to be initiated at high frequency. Expression vectors may include, but are not limited to, cloning vectors, modified cloning vectors, specifically designed plasmids or viruses.

The proteins of the present invention may exist as, but are not limited to, the complete proteins specified by the defined genes of the native protein or as any fragment or subunit thereof, or as hybrids of the complete protein or its fragments or subunits, so long as they retain platelet aggregation inhibiting activity. The complete proteins, as used herein, refers to the full length polypeptide produced by the appropriate genes. The complete proteins may be obtained by purification of the appropriate viper venom or by expression in an appropriate expression vector of the corresponding recombinant derived gene product. Protein fragments or subunits refers to any portion of the protein which contains fewer amino acids than the complete protein and retains the ability to inhibit platelet aggregation under the same conditions as does the native protein. Hybrid proteins include, but are not limited to, fusion proteins or proteins resulting from the expression of multiple genes within the expression vector. A fusion protein is defined as one in which a limited number of amino acids coded for by the expression vector are expressed and the expression results in their attachment to the specific platelet aggregation inhibiting polypeptide. Proteins resulting from multiple genes may include the specific polypeptide linked to a second polypeptide or peptides by peptide bonds that enhance inhibition of platelet aggregation.

Polypeptides within the scope of the present invention have been isolated from viper venom of Trimeresurus gramineus, Echis carinatus and Agkistrodon piscivorus, Bitis arietans and Eristocophis macmahonii. All of these polypeptides are potent platelet aggregation inhibitors.

The sequence for an inhibitor isolated from Echis carinatus venom is :

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Asp-Met-Asp-Asp-Tyr-Cys-Asn-Gly-Lys-Thr-
Cys-Asp-Cys-Pro-Arg-Asn-Pro-His-Lys-Gly-Pro-Ala-
Thr

This inhibitor is hereinafter referred to as Echistatin.
The sequence for an inhibitor isolated from Agkistrodon piscivorus venom is.

Val-Gln-Pro-Ala-Asn-Pro-Cys-Cys-Asp-Ala-Ala-Thr-
Cys-Lys-Leu-Thr-Pro-Gly-Ser-Gln-Cys-Ala-Glu-Gly-
Leu-Cys-Cys-Asp-Gln-Cys-Lys-Phe-Ile-Lys-Ala-Gly-
???-Ile-Cys-???-Arg-Ala-Arg-Gly-Asp-Asn...

The identities of certain amino acids are not known at this time, nor is the identity of the sequence following the 46th amino acid. The inhibitor is neverthless believed to fall within the scope of the present invention, and is hereinafter identified as Agkistrostatin.

One sequence for an inhibitor isolated from Bitis arietans venom is :

Ser-Pro-Pro-Val-Cys-Gly-Asn-Glu-Leu-Leu-Glu-Glu-
Gly-Glu-Glu-Cys-Asp-Cys-Gly-Ser-Pro-Ala-Asn-Cys-
Gln-Asp-Arg-Cys-Cys-Asn-Ala-Ala-Thr-Cys-Lys-Leu-
Thr-Pro-Gly-Ser-Gln-Cys-Asn-His-Gly-Glu-Cys-Cys-
Asp-Gln-Cys-Lys-Phe-Lys-Lys-Ala-Arg-Thr-Val-Cys-
Arg-Ile-Ala-Arg-Gly-Asp-Trp-Asn-Asp-Asp-Tyr-Cys-
Thr-Gly-Lys-Ser-Ser-Asp-Cys-Pro-Trp-Asn-His

The inhibitor is hereinafter identified as Bitistatin 1.

Another sequence for an inhibitor isolated from Bitis arietans venom is :

Ser-Pro-Pro-Val-Cys-Gly-Asn-Lys-Ile-Leu-Glu-Gln-
Gly-Glu-Asp-Cys-Asp-Cys-Gly-Ser-Pro-Ala-Asn-Cys-
Gln-Asp-Gln-Cys-Cys-Asn-Ala-Ala-Thr-Cys-Lys-Leu-
Thr-Pro-Gly-Ser-Gln-Cys-Asn-His-Gly-Glu-Cys-Cys-
Asp-Gln-Cys-Lys-Phe-Lys-Lys-Ala-Arg-Thr-Val-Cys-
Arg-Ile-Ala-Arg-Gly-Asp-Trp-Asn-Asp-Asp-Tyr-Cys-
Thr-Gly-Lys-Ser-Ser-Asp-Cys-Pro-Trp-Asn-His .

The inhibitor is hereinafter identified as Bitistatin 2.

Another sequence for an inhibitor isolated from Bitis arietans venom is :

Val-Ser-Pro-Pro-Val-Cys-Gly-Asn-Lys-Ile-Leu-Glu-
Gln-Gly-Glu-Asp-Cys-Asp-Cys-Gly-Ser-Pro-Ala-Asn-
Cys-Gln-Asp-Gln-Cys-Cys-Asn-Ala-Ala-Thr-Cys-Lys-
Leu-Thr-Pro-Gly-Ser-Gln-Cys-Asn-His-Gly-Glu-Cys-
Cys-Asp-Gln-Cys-Lys-Phe-Lys-Lys-Ala-Arg-Thr-Val-
Cys-Arg-Ile-Ala-Arg-Gly-Asp-Trp-Asn-Asp-Asp-Tyr-
Cys-Thr-Gly-Lys-Ser-Ser-Asp-Cys-Pro-Trp-Asn-His .

The inhibitor is hereinafter identified as Bitistatin 3.

The sequence for an inhibitor isolated from Eristocophis macmahonii venom is :

Gln-Glu-Glu-Pro-Cys-Ala-Thr-Gly-Pro-Cys-Cys-Arg-
Arg-Cys-Lys-Phe-Lys-Arg-Ala-Gly-Lys-Val-Cys-Arg-
Val-Ala-Arg-Gly-Asp-Trp-Asn-Asp-Asp-Tyr-Cys-Thr-
Gly-Lys-Ser-Cys-Asp-Cys-Pro-Arg-Asn-Pro-Trp-Asn-
His .

The inhibitor is herinafter identified as Eristostatin.

6

Another sequence for an inhibitor isolated from Bitis arietans venom is :

Ser-Pro-Pro-Val-Cys-Gly-Asn-Glu-Ile-Leu-Glu-Gln-
Gly-Glu-Asp-Cys-Asp-Cys-Gly-Ser-Pro-Ala-Asn-Cys-
Gln-Asp-Gln-Cys-Cys-Asn-Ala-Ala-Thr-Cys-Lys-Leu-
Thr-Pro-Gly-Ser-Gln-Cys-Asn-His-Gly-Glu-Cys-Cys-
Asp-Gln-Cys-Lys-Phe-Lys-Lys-Ala-Arg-Thr-Val-Cys-
Arg-Ile-Ala-Arg-Gly-Asp-Trp-Asn-Asp-Asp-Tyr-Cys-
Thr-Gly-Lys-Ser-Ser-Asp-Cys-Pro-Trp-Asn-His

This polypeptide, referred to as Bitistatin 4, is a potent inhibitor of platelet aggregation assessed in vitro, ex vivo and in vivo in a canine model of repetitive thrombus formation. Bitistatin 4 displayed dose-responsive antithrombotic effects with 10 μg/kg, i.v. being a treshold anti-aggregatory dose and 100 μg/kg i.v. exhibiting full inhibition of platelet aggregation.

The sequences identified above are specific examples of polypeptides falling within the sequence defined in formula I, and should not be interpreted as limiting the scope of the present invention. They have been defined by means of amino acid sequencing. It will be understood that natural allelic variations exist. These variations may be demonstrated by (an) amino acid difference(s) in the overall sequence or by deletions, substitutions, insertions, inversions or additions of (an) amino acid(s) in said sequence.

## EXAMPLE 1

## METHODS

Materials - The venom of Echis carinatus was purchased from Miami Serpentarium Laboratories, Salt Lake City, UT. Low molecular weight peptide standards, Sephadex G-50, and Mono S FPLC columns were from Pharmacia, Inc. Servalyt Precote isoelectric focusing gels were purchased from Serva Fine Biochemicals, Inc., and isoelectric focusing marker proteins were from BDH Chemicals, Ltd. C18 HPLC columns were the products of Vydac.

## Preparation of Platelets

Rabbit whole blood was collected from New Zealand white rabbits. Blood was centrifuged at 200 x g for 10 min. The supernatant, platelet rich plasma, was centrifuged for 15 min at 800 x g in the presence of apyrase (20 μg/ml) and PGE$_1$ (5 ng/ml). The pellet was resuspended in 10 ml of wash buffer (138 mM NaCl, 2.9 mM KCl, 0.31 mM Na$_2$HPO$_4$, 1 mM MgCl$_2$, 5 mM HEPES, pH 6.5, 5 mM glucose, 0.1% NaHCO$_3$, 1 mM EGTA and 0.35% bovine serum albumin). Apyrase was added to a final concentration of 20 μg/ml. The resuspended pellet was centrifuged at 800 x g for 15 min, then washed a second time in the same buffer. The washed platelets were then suspended in wash buffer, pH 7.4, with EGTA omitted at a concentration of 2-5 x 10⁸ cells/ml.

## Platelet Aggregation Assay

Platelet aggregation was measured at 37°C in an aggregometer. The reaction mixture contained washed platelets (2-5 x 10⁸ cells/ml), fibrinogen (100 μg/ml), Ca$^{2+}$ (1 mM), ADP (10 μM), epinephrine (2 μg/ml), and the platelet aggregation inhibitor fraction to be tested. The aggregation was initiated by adding ADP and epinephrine 1 min after the other components had been added. The reaction was allowed to proceed for at least 2 min. The extent of inhibition of aggregation was expressed as the percentage of aggregation observed in the absence of inhibitor.

## Purification of Echistatin

Rabbit platelet aggregation assays were used to monitor the activity during purification. Echis carinatus lyophilized venom (1g) was dissolved in 12 ml of 10 mM ammonium bicarbonate, pH 7.7 containing 20 mM DTT. After 15 min at room temperature, the solution was centrifuged at 45,000 x g for 45 min. The pellet was discarded and the supernatant was loaded onto a 2.5 x 125 cm column of Sephadex G-50 equilibrated and eluted at 4° with 10 mM MES, pH 5.3. Fractions containing platelet aggregation inhibitory activity were pooled and concentrated under vacuum. After desalting on a column of Sephadex G-15, 10-13 mg of protein were loaded onto a Mono S FPLC column which had been equilibrated with 10 mM MES, pH 5.3. The bound protein was fractionated at 4° with a linear gradient from 0 to 0.3 M NaCl to 10 mM MES, pH 5.3. The flow rate was 0.6 ml/min. Inhibitory activity eluted as a single peak at about 0.17 M NaCl. The pooled activity was loaded directly onto a C18 reverse phase HPLC column which had been equilibrated with 0.1% (v/v) trifluoroacetic acid in water. The column was developed at room temperature with a polyphasic acetonitrile gradient in 0.1% aqueous TFA, at a flow rate of 0.7 ml/min. Inhibitory activity eluted as a single peak at an acetonitrile concentration of 17%. Volatile material was removed by vacuum centrifugation followed by lyophilization. The resulting protein was homogeneous as judged by SDS polyacrylamide gel electrophoresis, isoelectric focusing, rechromatography on reverse phase HPLC, and N-terminal sequence determination. The yield of purified protein, which we have named Echistatin, was 2.2 mg from 1 g. of starting material.

## SDS Polyacrylamide Gel Electrophoresis

We used a modification of the Laemmli system (Nature, 1970, 227, pp. 680-685, hereby incorporated by reference). Stacking and separating gels (1.5 mm) contained 8% and 20% polyacrylamide, respectively. Urea (8 M) was added to the separating gel. The gels were run at 85 volts for 1.5 hr and then at 90 volts for 17 hr. Protein was detected by Coomassie brilliant blue R-250 staining.

## Isoelectric Focusing

A Servalyt Precote isoelectric focusing gel was used. Samples were run at a constant power of 1 W in an LKB Ultraphore apparatus. The gels were stained with Serva Blue W.

## Reduced and Carboxymelthylated Echistatin

Echistatin (0.75 mg protein) was reduced for 1 hr at room temperature with 20 mM DTT in the presence of 0.28 M Tris and 6.7 M guanidine hydrochloride, in a volume of 0.6 ml. Iodoacetic acid (0.06 ml) was then added to give a final concentration of 0.136 M and alkylation was allowed to proceed for 20 min at room temperature. The reduced, carboxymethylated Echistatin was isolated from reagents by C18 reverse phase HPLC.

## Chymotryptic Cleavage of Echistatin

Echistatin was digested with chymostrypsin at a ratio of substrate to protease of 40 :1 (w/w). The proteolytic reaction was carried out for 4 hr at 37°C in 0.2 M ammonium bicarbonate, pH 7.8. The reaction mixture was then lyophilized, and chymotryptic peptides were isolated by reverse phase HPLC.

## Amino Acid Sequence Determination

Purified Echistatin and selected chymotryptic fragments were subjected to automated protein sequence analysis in an Applied Biosystems 470A Sequencer using the sequencer program and reagents supplied by the manufacturer. Released amino acid derivatives were identified with the aid of an on-line HPLC system.

## Protein Determination

Protein was determined by the method of Lowry et al. (J. of Biol. Chem., 1951, 193, pp. 265-275, hereby incorporated by reference) using bovine serum albumin as standard.

## RESULTS

### Purification

Echistatin was purified to homogeneity from the soluble portion of the lyophilized venom of Echis carinatus in three chromatographic steps. Due to the presence of platelet aggregation stimulatory activity in the crude venom, the inhibitor could not be reliably detected in this fraction. Following gel filtration on Sephadex G-50, however, a single peak of inhibitory activity was observed. This activity was further purified by cation exchange FPLC on Mono S, and finally by C18 reverse phase HPLC.

One gram of lyophilized venom yielded 2.2 mg of purified Echistatin. This material exhibited a single band on SDS polyacrylamide gel electrophoresis in the presence of 8M urea and on isoelectric focusing. Rechromatography on reverse phase HPLC resulted in a single symmetrical absorbance peak which corresponded exactly with platelet aggregation inhibitory activity. Further confirmation of the homogeneity of the Echistatin preparation was obtained during amino acid sequencing of the native or the reduced and carboxymethylated protein. The only amino acid residue observed during the first sequencer cycle was glutamic acid.

## Molecular Weight and Isoelectric Point

The apparent molecular weight of reduced Echistatin during electrophoresis on 20% SDS polyacrylamide gels in the presence of 8 M urea was 5,800 daltons. This is similar to the value calculated from the amino acid sequence (see below). Under nonreducing conditions, the protein migrated somewhat more slowly, corresponding to an apparent molecular weight of 7,000 daltons. Surprisingly, when native Echistatin was treated with hydroxyethyl disulfide, the apparent molecular weight was only about 4,000 daltons. A minor band, probably resulting from incomplete derivatization, was observed in the hydroxyethyl disulfide-treated Echistatin.

Both native and disulfide-treated Echistatin exhibited on pl of 8.3. Upon treatment with dithiothreitol, a minor band with a pl of 7.5 was detected in addition to the predominant band at pl 8.3.

## Amino Acid Composition

The amino acid composition of Echistatin, obtained after 20 hr of hydrolysis of the reduced, carboxymethylated protein, is shown in Table I. The composition obtained by this analysis was compared with that predicted from the sequence (see below). Most values obtained by these two methods agreed closely. The content of cysteinyl residues found by amino acid composition (6.7) was lower than that predicted by sequence (8). This may have been due to incomplete carboxymethylation of Echistatin.

## TABLE I

Comparison of the amino acid composition of Echistatin between the value determined after hydrolysis and that calculated from sequence :

## TABLE I

| Amino Acid | Residues per molecule | |
|---|---|---|
| | Acid hydrolysis | Sequence |
| Cysteine | 6.5 | 8 |
| Aspartic acid 1 | 8.4 | 8 |
| Threonine | 2.8 | 3 |
| Serine | 1.2 | 1 |
| Glutamic acid 2 | 3.3 | 3 |
| Proline | 4.2 | 4 |
| Glycine | 5.3 | 5 |
| Alanine | 1.8 | 2 |
| Methionine | 0.6 | 1 |
| Isoleucine | 1.0 | 1 |
| Leucine | 1.1 | 1 |
| Tyrosine | 1.0 | 1 |
| Phenylalanine | 1.0 | 1 |
| Histidine | 1.2 | 1 |
| Lysine | 5.2 | 5 |
| Arginine | 4.1 | 4 |
| Total | 48.7 | 49 |

[1] The value includes asparagine.
[2] The value includes glutamine.

### Effect of Echistatin on platelet aggregation in vitro

Platelet aggregation studies indicate that Echistatin is more potent than trigramin in inhibiting human gel filtered platelet aggregation induced by ADP in the presence of 0.1 M mg/ml fibrinogen ($IC_{50}$=30 nM vs. 150 nM).

Echistatin inhibits aggregation of human platelets induced by ADP collagen, platelet activating factor or epinephrine at doses 30-300 nM.

### Human Platelet Rich Plasma

Blood was drawn into 0.1 vol. of 3.8% sodium citrate by venipuncture from normal human volunteers. Platelet rich plasma (PRP) was prepared by centrifugation at 400 x g for 12 min. Aggregations were performed by adding ADP collagen, platelet activating factor or epinephrine to PRP and measured in a Sienco aggregometer.

### Human Gel Filtered Platelets

Blood was drawn into 0.1 volume of acid-citrate-dextrose (85 mM sodium citrate, 64 mM citric acid, 110 mM dextrose) by venipuncture from normal human volunteers. Platelet rich plasma was prepared by centrifugation at 400 x g for 12 min. PGE₁ (5 µg/ml) was added and platelets were collected by centrifugation at 800 x g for 12 min. The platelet pellet was resuspended into human platelet buffer (140 mM NaCl, 7.9 mM KCl, 3.2 mM Na₂HPO₄, 6 mM HEPES, 2% bovine serum albumin, 0.1% dextrose, pH 7.2) and filtered over Sepharose 2B that was previously equiliberated in human platelet buffer. Platelets were counted and adjusted to 2 x 10⁸/ml with human platelet buffer.

Amino Acid Sequence

The amino acid sequence of Echistatin was obtained by automated Edman degradation of the reduced, carboxymethylated protein. The protein has 49 amino acids with an N-terminal glutamic acid residue and a C-terminal threonine, and is shown above. Cysteine comprised 8 residues, or greater than 16%, of the total amino acids in Echistatin. The sequence was repeated two times with the same result. When native Echistatin was sequenced, no PTH-amino acid peaks were observed during sequencer cycles where cysteinyl residues were expected, further confirming the assignments at these residues. The disulfide status of these cysteinyl residues is not known.

Further confirmation of the sequence was obtained by analysis of chymotryptic peptides isolated from the reduced, carboxymethylated protein (Table II).

TABLE II

Amino Acid Composition of Chymotryptic Peptides of Echistatin

The values in parentheses are the numbers of amino acid residues determined by sequence.

## TABLE II

|          | C1      | C2      | C3      | C4      | C3 +C4  |
|----------|---------|---------|---------|---------|---------|
| Cys (CM) | 2.6(4)  | 0.7(1)  | 1.3(3)  |         | 1.5(3)  |
| Asx      | 1.3(1)  |         | 2.7(3)  |         | 2.9(3)  |
| Thr      |         | 1.0(1)  | 1.0(1)  | 1.0(1)  | 1.4(2)  |
| Ser      | 1.1(1)  |         |         |         |         |
| Glu      | 2.0(2)  | 1.1(1)  |         |         |         |
| Gly      | 1.2(1)  | 1.4(1)  | 1.9(1)  | 1.2(1)  | 2.2(2)  |
| Ala      |         |         |         | 1.0(1)  | 0.8(1)  |
| Ile      |         | 0.6(1)  |         |         |         |
| Leu      |         | 0.9(1)  |         |         |         |
| His      |         |         | 1.2(1)  |         | 1.3(1)  |
| Phe      | 0.9(1)  |         |         |         |         |
| Lys      | 1.1(1)  | 1.9(2)  | 1.7(1)  | 1.0(1)  | 2.2(2)  |
| Arg      | 1.2(1)  | 0.7(1)  | 1.1(1)  |         | 1.1(1)  |
| Pro      | 1.3(1)  |         | 1.4(2)  | 1.0(1)  | 2.5(3)  |

Echistatin contains the sequence Arg-Gly-Asp which is common to certain proteins which bind to the glycoprotein IIb/IIIa complex on the platelet surface, at residues 24-26. This sequence is part of a putative platelet binding site on the fibrinogen molecule.

Echistatin exhibited unusual behavior during SDS polyacrylamide gel electrophoresis in the presence of 8M urea. The native protein migrated with an apparent molecular weight of 7,000 daltons, compared with the value of 5,400 daltons obtained from sequence analysis. Under nonreducing conditions, proteins with intrachain disulfide bonds generally bind less than expected amounts of SDS, while prior reduction increases SDS binding to levels seen with proteins lacking intrachain disulfide bonds (Pitt-Rivers et al., Biochem J., 1968, 109, pp. 825-830). Thus the increased mobility of reduced Echistatin during SDS polyacrylamide gel electrophoresis suggests that this protein contains one or more intrachain disulfide bonds in its native conformation.

Treatment of Echistatin with 80 mM dithiothreitol at room temperature for 20 min completely destroyed its inhibitory activity towards platelet aggregation. Under similar conditions, 80 mM hydroxyethyl disulfide treatment led to the loss of 50% of the inhibitory activity. These results, like those of the SDS gel electrophoresis studies, imply that one or more intrachain disulfide bonds play an important role in maintaining the conformation of Echistatin necessary for its inhibitory activity.

## EXAMPLE 2

Methods of protein purification and determination used in Example 1 were employed to isolate and identify a platelet aggregation inhibitor present in Agkistrodon piscovorus, venom. The protein, whose amino acid sequence is identified above, displayed characteristics of a platelet aggregation inhibitor.

## EXAMPLE 3

Methods of protein purification and determination used in Example 1 were employed to isolate and identify a platelet aggregation inhibitor present in Bitis arietans venom. The protein, whose amino acid sequence is identified above, displayed characteristics of a platelet aggregation inhibitor.

## EXAMPLE 4

Methods of protein purification and determination used in Example 1 were employed to isolate and identify a second platelet aggregation inhibitor present in Bitis arietans venom. The protein, whose amino acid sequence is identified above, displayed characteristics of a platelet aggregation inhibitor.

## EXAMPLE 5

Methods of protein purification and determination used in Example 1 were employed to isolate and identify a third platelet aggregation inhibitor present in Bitis arietans venom. The protein, whose amino acid sequence is identified above, displayed characteristics of a platelet aggregation inhibitor.

## EXAMPLE 6

Methods of protein purification and determination used in Example 1 were employed to isolate and identify a platelet aggregation inhibitor present in Eristocophis macmahonii venom. The protein whose amino acid sequence is identified abhove, displayed characteristics of a platelet aggregation inhibitor.

Purification of various polypeptides within formula I to chemical homogeneity according to the present invention has permitted amino acid sequencing of the molecules. Based upon the amino acid sequence shown in formula I, one may advantageously prepare a synthetic gene corresponding to a disclosed amino acid sequence and introduce that gene into an appropriate host by appropriate cloning vectors. Alternatively, it is contemplated that the pure polypeptide may be prepared by obtaining the natural gene from venom producing cells, followed by recombination and cloning. It is therefore understood that the scope of the invention is not merely limited to polypeptides isolated by following the chromatographic procedures disclosed herein, or polypeptides falling within the formula I, but also includes these polypeptides as they may be prepared by genetic engineering techniques.

## EXAMPLE 7

### DNA and Plasmid Construction, and Protein Expression for Recombinant-leu 28-Echistatin

All enzymes and DNA molecular weight markers were from New England Biolabs. Dideoxy DNA sequencing kits were purchased from United States Biochemical Corporation Inc. Competent E. coli Jm109 was purchased from Stratagene. All the other reagents were analytical or electrophoresis grades.

All oligodeoxynucleotides were synthesized on an Applied Biosystems 380B DNA synthesizer using methylphosphoramidite. After deprotection with concentrated ammonia, oligodeoxynucleotides were desalted on a Pharmacia NAP-10 gel filtration column.

All oligodeoxynucleotides were phosphorylated by ATP and T4 polynucleotide kinase except the two oligodeoxynucleotides overhanging on 5′ ends of both strands. The kinased complementary oligodeoxynucleotides were heated to 95°C and then annealed by cooling slowly to room temperature. Ligation of the 3 duplexes was carried out at 14°C for 15 hours in a reaction mixture containing 0.5 nmol of each duplex, 50 mM Tris-HCl, pH 7.8, 10 mM MgCl$_2$, 20 mM dithiothreitol, 1 mM ATP, 50 µg/ml bovine serum albumin, and 4,000 units of T4 DNA ligase in a total volume of 130 µl. The ligation mixture was fractionated by 10% native polyacrylamide gel electrophoresis. The assembled leu 28-echistatin gene was cut from the polyacrylamide gel and electro-eluted. The eluted leu 28-echistatin gene was kinased, extracted by phenol/chloroform, and precipitated by ethanol before it was inserted into the expression vector.

Construction of pJC264 Expression Vector

A 714 bp HindIII-PstI fragment from pLCI-28 (obtained from B. Bachmann, E. coli Genetic Stock Center) containing the 3′-436 bp of the cheB gene and the 5′-263 bp of cheY gene (Matsumura et al., J. Bact., 160 (1984) pp. 36-41) was cloned into the pUC13 HindIII and PstI sites. In order to move the unique EcoRI site in the polylinker sequence of pUC13 adjacent to the cheY gene, polylinker sequences between the PstI and EcoRI sites were deleted. The resultant expression vector pJC264 contains a unique EcoRI site which allows genes of interest to fuse with the cheY gene. For other purposes, the HindIII site connecting pUC13 and the cheB gene was converted to a BamHI site by filling in with the Klenow fragment of E. coli DNA polymerase I and ligation with a synthetic BamHI linker.

The synthetic recombinant leu 28-echistatin gene was inserted into the EcoRI site of pJC264 by ligating one third of synthetic leu 28-echistatin gene prepared above with 0.1 ug EcoRI cut pJC264 under the following conditions : 50 mM Tris-HCl, pH 7.8, 10 mM MgCl$_2$, 20 mM dithio-threitol, 1 mM ATP, 50 ug/ml bovine serum albumin, and 800 units of T4 DNA ligase in a total volume of 20 ul. The ligation was carried out for 15 hours at 14°C.

Transformation of the Recipient Bacteria and Identification of the Positive Clones

One third of the pJC264 expression vector containing the inserted r-leu 28-echistatin gene (7 ul) was used to transform 50 ml of JM109 competent cells under standard conditions. The clones containing the r-leu 28-echistatin gene were selected by restriction mapping. The presence of the coding sequence of echistatin was verified by dideoxy sequence analysis.

Cell Growth and Preparation of Fusion Protein Pellet

Cultures were inoculated with an overnight seed culture (1% V/V) and grown in LB medium containing 100 ug amipicillin/ml. When the cell density reached an OD$_{600}$ reading of 0.2 to 0.3, IPTG was added to give a final concentration of 1 mM. Cells were harvested by centrifugation after another 10 h growth. The cell pellet from a 3000 mlculture was suspended in water to a final volume of 50 ml and sonicated at 4°C for 10 min. using a Fisher Sonic Dismembrator, Model 300, with the large tip at maximum power. The sonicated mixture was centrifuged at 20,000 g for 15 min. The pellet was resuspended in 8 ml water and layered onto 50% sucrose in 15 mM Tris-HCl, pH 7.4, and 0.15 M NaCl. Following centrifugation at 13,000 rpm for 30 min. in an SW28 Beckman rotor at 6°C, the pellet containing the insoluble CheY-Leu 28-echistatin fusion protein was resuspended in 6 ml water.

Cleavage of Fusion Protein

The fusion protein-containing pellet from a 1000 ml culture was cleaved with 60 mM cyanogen bromide in 70% formic acid in a total volume of 13 ml. The reaction proceeded for 15 hours at room temperature. The reagents were removed by extensive dialysis and lyophilization.

Purification and Refolding of Recombinant-leu 28-Echistatin

The cleaved fusion protein was first denatured with 8 M guanidine-HCl at 60°C for 5 min., and then reduced with 0.15 M dithiothreitol at room temperature for 10 min. in the presence of 0.35 M tris-base and 6 M guanidine-HCl. The reduced Leu 28-echistatin was purified by C18 reverse phase HPLC with a 0-65% acetonitrile gradient in the presence of 0.1% trifluoroacetic acid. The HPLC purified r-leu 28-echistatin was oxidatively refolded in 0.1 M ammonium acetate at a protein concentration of 0.2 mg/ml. The refolding reaction proceeded for 72 hours at room temperature with vortex mixing about once every 12 hours.

Platelet Aggregation Assay

Platelet aggregation was measured by the increase in light transmission. Briefly, r-leu 28-echistatin was added to the reaction mixture containing gel-filtered human platelets ($2 \times 10^8$ cells/ml), fibrinogen (100 µg/ml), and $Ca^{++}$ (1 mM). Platelet aggregation was initiated by adding ADP (final concentration of 10 µM) 1 min. after the other components had been added. The rate or extent of aggregation was expressed as the percentage of the maximal light transmission obtained in the absence of echistatin.

Flowchart for the Production of Recombinant leu 28-Echistatin

E. coli (harboring plasmid pJC264-echistatin)

Induce w/IPTG
Cell harvest
Cell lysis
Centrifugation

Fusion protein pellet

Cyanogen bromide

CnBr-cleaved fusion protein pellet

Denature (guanidine-HC1)
Reduce (dithiothreitol)

Fully-reduced, cleaved fusion protein pellet

C18 reverse phase HPLC

Fully reduced r-leu 28-echistatin

Reoxidation/refolding
C18 reverse phase HPLC

Purified, oxidized r-leu 28-echistatin

Design and Construction of Synthetic Gene

Native echistatin is a single chain polypeptide of 49 amino acid residues with a pI of 8.3. A synthetic r-leu 28-echistatin gene was designed and fused with the E. coli cheY gene. The primary structures of the platelet aggregation inhibitors isolated from other sources of the venom of vipers have revealed that methionine 28 is not a conserved amino acid in this family of inhibitors (Huang et al., (1987) J. Biol. Chem., 262, pp. 16157-16163). Therefore, methionine 28 of echistatin was replaced by a leucine and a methionine was inserted between the cheY protein and recombinant-leu 28-echistatin to generate a cyanogen bromide cleavage site. Two EcoRI sticky ends were designed on the flanking ends in order to allow a junction to the expression vector. A stop codon immediately preceding the 3'-flanking EcoRI site was inserted. The synthetic r-leu 28-echistatin gene was assembled from three double strand fragments which contain KpnI and AvaI restriction sites in the joining sites. Preferred E. coli codons were used in the structural gene except where restriction sites were purposely created. The constructed gene was verified by restriction mapping and DNA sequence analysis.

## Construction of Expression Vector

In order to express r-leu 28-echistatin in E. coli, the expression vector pJC264 was used. The vector contains the gene encoding the amino-terminal 88 amino acids of the E. coli cheY protein, previously shown to be abundantly and stably expressed in E. coli (Matsumura et al., (1984), J. Bact., 160, pp. 36-41). CheY gene expression in this vector is controlled by E. coli lac promoter-operator from pUC13 (Messing, Methods in Enzymology, Wu et al., eds Academic Press, New York, 101, pp. 20-78 (1983)). Following the cheY gene in pJC264, unique PstI and EcoRI sites facilitate the cloning of new open reading frames and their inducible expression as cheY fusion proteins. The synthetic r-leu 28-echistatin gene was inserted into the EcoRI site of pJC264.

This vector has several advantages over existing expression systems. CheY fusions are often expressed at high levels (see below and Bailey, et al., (1987) J. Indust. Micro., 2, pp. 47-52). The vector is derived from the high copy number plasmid pUC13 (Messing, Methods in Enzymology, Wu, et al. eds, Academic Press, New York, 101, (1983) pp. 20-78) and efficient replication may contribute to the high expression levels observed. CheY fusion proteins are often found as insoluble inclusion bodies. This insolubility can be exploited in the purification of the fusion protein. The insolubility of the protein may also prevent its degradation by intracellular proteases. Because of the small size of the CheY moiety, the mass ratio of the protein of interest is higher than would be achieved by fusion to the larger, more commonly used $\beta$-galactosidase. Finally, the absence of cysteine residues in the CheY protein circumvents potential problems of incorrect disulfide bond formation in the fusion protein.

## Expression of Recombinant-leu 28-Echistatin

Expression of recombinant-leu 28-echistatin in E. coli JM109 was induced by IPTG as previously described. The expression product at different times after induction was analyzed by 14% SDS polyacrylamide gel electrophoresis. After 2 hours of induction, a polypeptide of the expected molecular weight of the cheY-echistatin fusion protein (14.5 kDa) was detected in the total cellular lysate. At 6 hours of induction, this protein accumulated to a maximum level accounting for at least 20% of total cell protein. The expressed protein was insoluble after sonication of the cells, suggesting that inclusion bodies were formed during the expression. The sonicated pellet was subjected to cyanogen bromide cleavage for isolation of recombinant-leu 28-echistatin.

## Purification and Renaturation of Recombinant-leu 28-Echistatin

The cyanogen bromide cleaved proteins were reduced by an excess of dithiothreitol in the presence of 6 M guanidine-HCl before purification. Fractionation of the reduced proteins by reverse phase HPLC revealed a peak which had a retention time similar to that of reduced native echistatin. Amino acid sequence analysis of the protein from this peak showed that it contained recombinant-leu 28-echistatin with a purity of more than 90%. Since the reduced echistatin was in 0.1% trifluoroacetic acid (about pH 2) during HPLC, thiol-disulfide exchange reactions within recombinant-leu 28-echistatin were expected to be very slow. Therefore, the lyophilized echistatin was directly refolded in the presence of 0.1 M ammonium acetate without further denaturation and reduction. It has been proposed that renaturation of an inactive protein is facilitated by adding a thiol/disulfide mixture to the renaturation solution (Tam et al., Nature, 309 (1984) pp. 376-378). We have renatured recombinant-leu 28-echistatin with 0.5 mM dithiothreitol and 1 mM oxidized dithiothreitol in the presence of 6 M guanidine-HCl. However, the yield of refolded echistatin was not increased under such conditions. Correctly folded echistatin was isolated from the unfolded species by reverse phase HPLC. The HPLC profile indicated that more than 30% of the reduced echistatin was correctly folded under the given conditions. The amount of correctly folded pure echistatin obtained by the purification procedure was estimated about 1.5 mg per liter cell culture. Recombinant-leu 28-echistatin was eluted from C18 column at a slightly higher concentration of acetonitrile than native echistatin. This may be the result of replacing methionine 28 in native echistatin with leucine in recombinant-leu 28-echistatin. The N-terminal amino acid sequence of recombinant-leu 28-echistatin was determined by automated Edman degradation. The sequence up to residue 32 from the N-terminus was found to be identical to the native one except for the leucine to methionine substitution at residue 28.

Biological Activity of Recombinant-leu 28-Echistatin

A comparison of biological activity of recombinant-leu 28- and native echistatin is shown in Fig. 5. The inhibition of platelet aggregation by echistatin was measured in the presence of human fibrinogen and $CaCl_2$ after adding ADP. Like native echistatin, the recombinant protein did not affect the initial decrease in light transmittance following addition of ADP, suggesting that the inhibitors did not influence platelet activation. Recombinant-leu 28-echistatin and native echistatin inhibited platelet aggregation, measured as either rate or extent of aggregation, with the same $IC_{50}$. Therefore, recombinant-leu 28-echistatin appears to be identical to native echistatin in its ability to affect platelet aggregation. The results suggest that the presence of methionine at position 28 in native echistatin is not essential for the correct folding and biological activity of this platelet aggregation inhibitor.

The potential exists, in the use of recombinant DNA technology, for the preparation of derivative polypeptides variously modified by resultant single or multiple amino acid substitutions, deletions, additions or replacements, for example, by means of site directed mutagenesis of the underlying DNA. All such allelic variations and modifications resulting in derivative polypeptides are included within the scope of this invention so long as the essential, characteristic platelet aggregation inhibiting activity remains unaffected in kind. The polypeptides are prepared (1) having methionine as its first amino acid (present by virtue of the ATG start signal codon insertion in front of the structural gene) or (2) where the methionine is intra- or extracellularly cleaved, having its normally first amino acid, or (3) together with either its signal polypeptide or a conjugated protein other than the conventional signal polypeptide, the signal polypeptide or conjugate being specifically cleavable in an intra- or extracellular environment (see British Patent Application Publication No. 2,007,676A), or (4) by direct expression in mature form without the necessity of cleaving away any extraneous, superfluous polypeptide. The latter is particularly important where a given host may not, or not efficiently, remove a signal peptide where the expression vehicle is designed to express the protein together with its signal peptide. In any event, the thus produced platelet aggregation inhibitor in its various forms, is recovered and purified to a level fitting it for use in the treatment of various vascular conditions or diseases.

Synthetic Polypeptides of Formula I

Polypeptides of the invention may be prepared using solid phase synthesis, such as that described by Merrifield, J. Am. Chem. Soc., 85, 2149 (1964), although other equivalent chemical syntheses known in the art can also be used, such as the syntheses of Houghten, Proc. Natl. Acal. Sci., 82, 5132 (1985). Solid-phase synthesis is commenced from the C-terminus of the peptide by coupling a protected amino acid to a suitable resin, as generally set forth in U.S. Pat. No. 4,244,946, issued Jan. 21, 1982 to Rivier et al., the disclosure of which is incorporated herein by reference. Examples of synthesis of this general type are set forth in U.S. Pat. Nos. 4,305,872 and 4,316,891. Discussion of the solid-phase synthesis of a 41-residue polypeptide is set forth in Science, 213, 1394-1397 (September 1981) in an article by Vale et al., which refers to a more detailed discussion of the synthesis, which appears in an aritcle by Marke et al. in J. Am. Chem. Soc., 103, 3178 (1981).

In synthesizing the polypeptides, the carboxyl terminal amino acid, having its alpha-amino group suitable protected, is coupled to a chloromethylated polystyrene resin or the like. After removal of the alpha-amino protecting group, as by using trifluoroacetic acid in methylene chloride, the next step in the synthesis is ready to proceed. Other standard cleaving reagents and conditions for the removal of specific amino protecting groups may be used, as described in the open literature.

The remaining alpha-amino- and side-chain-protected amino acids are then coupled stepwise in the desired order to obtain an intermediate compound connected to the resin. As an alternative to adding each amino acid separately in the synthesis, some of them may be coupled to one another prior to the addition to the growing solid-phase chain. The selection of the appropriate coupling reagents is within the skill of the art.

Common to chemical syntheses of peptides is the protection of the labile side-chain groups of the various amino acid moieties with suitable protecting groups at that site until the group is ultimately removed after the chain has been completely assembled. Also common is the protection of the alpha-amino group on an amino acid or a fragment while that entity reacts at the carboxyl group followed by the selective removal of the alpha-amino-protecting group to allow subsequent reaction to take place at that location. Accordingly, it is common that, as a step in the synthesis, an intermediate compound is produced which includes each of the amino acid residues located in the desired sequence in the peptide chain with various of these residues having side-chain protecting groups. These protecting groups are then commonly removed substantially at the same time so as to produce the desired resultant product following purification.

After the desired amino-acid sequence has been completed, the intermediate peptide is removed from the resin support by treatment with a reagent, such as liquid HF, which not only cleaves the peptide from the resin, but also cleaves all the remaining side-chain protecting groups. The polypeptide can then be purified by gel permeation followed by semipreparative HPLC, as described in Rivier et al., Peptides : Structure and Biological Function (1979) pp. 125-128. A purity of at least 93% or higher (based upon all peptides present) is reasonably obtainable and is preferred for clincial testing and/or use. Purity of 98% is practical ; however, for certain in vitro applications, lower purity may be acceptable. Accordingly, the polypeptide is considered useful when it is in substantially pure form which, for purposes of this application, means at least about 50 weight percent, based upon all peptides present.

Examples of various polypeptides of the present invention and methods of synthesis are presented below.

## EXAMPLE 8

Boc-O-benzylthreonine-PAM resin, Boc protected amino acids and all other reagents required for synthesis on the Applied Biosystems 430 A (ABI) peptide synthesizer were obtained from the manufacturer. Sidechain protected Asp, Glu and His were supplied by Bachem, Inc. The solvents dimethylformamide (DMF) and methylene chloride ($CH_2Cl_2$) were obtained from Burdick and Jackson. Dithiothreitol (DTT) was purchased from Bethesda Res. Labs. Dithioerythritol (DTT) was obtained from Chemical Dynamics. p-cresol and p-thiocresol were obtained from Aldrich Chemical Co.

## Echistatin Synthesis

Starting with 0.50 mM (0.69 g) of Boc-Thr(Bzl)O-Pam-resin (substitution at 0.72 mM of Thr/g of resin) the synthesis was carried out in a stepwise manner using the ABI automated peptide synthesizer. Kent and Clark-Lewis (1985) Synthesic Peptides in Biology and Medicine, Proc. Labsystems Res. Symp., Eds., Alitalo et al., Elsevier, Netherlands pp. 29-57. The amino acids were introduced using the manufacturer's prepacked cartridges (2 mM each). Sidechain protection was Arg (Tos), Asp (OcHx), Cys (Meb), Glu (OcHx), His (Bom) Lys[Z(Cl)], Ser (Bzl), Thr (Bzl), Tyr[Z(Br)]. [Tos, Tosyl ; cHx, cyclo-hexyl ; Meb, 4-methylbenzyl ; Z(Cl), 2-chlorobenzyloxycarbonyl ; Bom, benzyloxymethyl ; Bzl, benzyl ; Z(Br), 2 bromobenzyloxycarbonyl]. Double coupling with symmetric anhydrides (performed in $CH_2Cl_2$ followed by solvent exchange with DMF) were used for all Boc-protected amino acids except for Arg (Tos), Asn and His (Bom), where hydroxybenzotriazole esters in DMF were used in a double coupling protocol. In order to protect against undesired acid-catalyzed oxidations of Cys and Met (Draper et al. (1973) J. Med. Chem. Vol. 16, pp. 1326-1329) during trifluoroacetic acid (TFA) deblocking, 0.1% (wt/v) DTE was added as a scavenger. Following the coupling of N-terminal Glu the Boc group was removed using TFA and the peptide-resin was dried. The final weight of N-terminal deblocked peptide-resin was 4.15 g.

## HF Cleavage and Oxidation

The assembled peptide-resin (2.0 g) was suspended in a mixture of 3 ml of 1 :1 (v :v) p-thiocresol/p-cresol in an HF apparatus (Peninusula Labs. Inc., Type 1B). The system was evacuated with a mechanical vacuum pump and HF condensed (30 mL) using liquid nitrogen cooling. After stirring at 0-5°C for 1 1/2 hr the reaction mixture was evaporated in vacuo using a liquid nitrogen trap (20-30 min). The residue was triturated with ether, filtered and washed 3 times with additional ether. The filtered residue was immediately transferred to 4 liters of a stirred solution of dilute acetic acid (0.4%/$H_2O$). After stirring for several minutes the pH of the mixture was adjusted to 8.0 with ammonium hydroxide. Following filtration to remove resin, the crude oxidation product was maintained without stirring at 5°C (18 hr) and subsequently at ambient temperature (19-20°C) for 3 days. Analytical HPLC was used to monitor the progress of the oxidation. A qualitative Ellman test (Habeeb, Methods in Enzymology (1972), Eds. Hirs and Timasheff, Academic Press New York pp. 457-464) was used to monitor the disappearance of free sulfhydryl groups before proceeding with purification. This test was performed on a 1 ml sample which was lyophilized in order to remove residual p-thiocresol.

## Purification of Crude Oxidized Echistatin

The crude oxidized solution (4 l) was acidified by addition of acetic acid (10 ml) and pumped directly onto

a $C_{18}$-silica (5 x 30 cm, 15 m, 300 A) cartridge (Waters Associates). The product was purified using preparative HPLC (Separations Technology, Inc.). A step gradient (100 mL increments) which was generated from 1 liter each of successively increasing concentrations of mobile phase. A flow rate of 70 mL/min was used to elute the product. Homogenous (>95%) fractions as determined by RP-HPLC (Vydac $C_{18}$, 218TP5415) were pooled and lyophilized to give 72 mg of product. The semi-pure product was contaminated with a less polar component as a shoulder to the product peak. The product was further purified by repassage on HPLC in the same manner as described above to yield echistatin (54 mg). Based on 0.25 mM of starting resin this weight represents a 4 percent overall yield. Homogeneity was demonstrated by analytical HPLC. Coinjection of synthetic product with native material gave a single peak. Product was further characterized by amino acid analysis after hydrolysis with 6N HCl (Table 3) and by automated Edman degradation (ABI 470A Protein Sequencer).

## TABLE 3

### AMINO ACID ANALYSIS[a] OF SYNTHETIC ECHISTATIN
### (THEORETICAL NUMBER IN PARENTHESIS)

| | | | |
|---|---|---|---|
| Lys | 5.00 (5) | Gly | 5.03 (5) |
| His | 1.00 (1) | Ala | 2.10 (2) |
| Arg | 3.87 (4) | Cys[d] | 7.67 (8) |
| Asp | 8.13 (8) | Met[c] | 0.84 (1) |
| Thr[b] | 2.99 (3) | Leu | 0.98 (1) |
| Ser[b] | 1.04 (1) | Phe | 0.95 (1) |
| Glu | 3.07 (3) | Tyr | 1.00 (1) |
| Pro | 4.08 (4) | Ile + allo-Ile | 0.99 (1) |

[a]After hydrolysis with 6N HCl at 100°C for 70 hrs.
[b]Corrected for decomposition during hydrolysis.
[c]Uncorrected.
[d]Determined as cysteic acid following performic acid oxidation.

A maximum of 1.9 percent preview was observed. The high yield of PTH amino acids from the first step also demonstrated that cyclization of the C-terminal Glu to pyro-Glu had not occurred.

Reduction and Refolding of Synthetic Echistatin

Synthetic echistatin (0.50 mg) was dissolved in 1 ml of 0.07 M pH 8.0 ammonium acetate (10 mM DTT) and the course of reduction followed by analytical HPLC. After 1 hr the starting material was converted quantitatively to a single reduced product. Dialysis (24 hr) of the reduced product using a 12 mm diameter, 1000 MW cutoff, cellulose tubing (Spectrum Medical Ind.) against (4 l) of a 0.07 M ammonium acetate buffer (pH 8.0) produced only echistatin. In order to demonstrate that the echistatin was in its fully reduced form prior to reoxidation, the DTT reduction of synthetic echistatin was repeated as described but in the presence of 6M guanidine hydrochloride. Analytical HPLC confirmed that the reduced products had identical times. Isolation of reduced echistatin, by semi-preparative HPLC, followed by quantitative Ellman analysis (Habeeb, ibid.) showed the product to be in the octahydro form.

Platelet aggregation assay

Platelet aggregation was measured at 37°C in a chronolog aggregometer. The reaction mixture contained washed platelets (2 x $10^8$/ml), fibrinogen (100 mg/mL), $Ca^{2+}$ (1 mM), and the platelet aggregation inhibitor fraction to be tested. The aggregation was initiated by adding 10 mM ADP 1 min after the other components had been added. The reaction was allowed to proceed for at least 2 min. The extent of inhibition of aggregation was expressed as the percentage of the rate of aggregation observed in the absence of inhibitor. Synthetic echistatin possesses chemical and biological properties indistinguishable from those of natural echistatin (Table 4).

## TABLE 4

### EFFECT OF NATIVE ECHISTATIN AND SYNTHETIC ECHISTATIN ON INHIBITION OF HUMAN PLATELET AGGREGATION

| PEPTIDE | $IC_{50}(\mu M)(95\%$ CONFIDENCE LIMITS) |
|---|---|
| Native Echistatin | 0.032 (0.026-0.039) |
| Synthetic Echistatin | 0.033 (0.026-0.041) |

The concentration of peptide necessary to inhibit the rate of human platelet aggregation by 50% ($IC_{50}$) was determined using platelets from 2-4 donors. Determinations were performed in duplicate for each donor.

### EXAMPLES 9-20

Following the general synthesis procedures for preparing echistatin described in Example 8, many polypeptides having sequences similar to echistatin and falling within the general formula I were prepared.

## TABLE 5

### ECHISTATIN ANALOGS PREPARED BY SYNTHETIC MEANS, AND THEIR EFFECT ON HUMAN PLATELET AGGREGATION

| Example No. | Sequence | $IC_{50}(\mu M)$ (95% CONFIDENCE LIMITS) |
|---|---|---|
| 9 | phe 27-echistatin | .013 (.010-.016) |
| 10 | phe 27, leu 28-echistatin | .016 (.011-.022) |
| 11 | trp 27-echistatin | .017 (.014-.019) |
| 12 | pro 23, phe 27, leu 28-echistatin | .026 (.021-.033) |
| 13 | leu 28-echistatin | .034 (.026-.043) |
| 14 | phe 28-echistatin | .038 (.026-.058) |
| 15 | asn 22, phe 27, leu 28-echistatin | .038 (.031-.047) |
| 16 | (1-41)-echistatin amide | .066 (.048-.084) |
| 17 | (1-43)-echistatin amide | .081 (.066-.099) |
| 18 | met SO 28-echistatin | .088 (.033-.154) |
| 19 | (1-39)-echistatin amide | .327 (.214-.414) |
| 20 | (1-39)-echistatin | .800 (.580-1.1) |

The abbreviations above define the various synthesized polypeptides. In examples 9-15 and 18, the sequence position number(s) which is (are) substituted and the residue(s) replacing the naturally occuring residue(s) is (are) indicated (e.g. in Example 9, Asp, the residue at position 27 which occurs in natural echistatin, is replaced with Phe). Residues 1-26 and 28-49 are identical to natural echistatin. Examples 16, 17, 19 and 20 are sequences having residues toward the carboxyl terminal end omitted (e.g. in Example 20, residues 42-49 have been omitted). met SO in Example 18 represents methionine sulfoxide.

Therapeutic Utility

Polypeptides of the invention may be administered in any situation where inhibition of human or mammalian platelet aggregation or adhesion is desired.

Polypeptides of the invention are eliminated from circulation rapidly and are particularly useful in inhibiting platelet aggregation in situations where a strong antithrombotic of short duration of effectiveness is needed. Thus, they may find utility in surgery on peripheral arteries (arterial grafts, carotid endarterectomy) and in cardiovascular surgery where manipulation of arteries and organs, and/or the interaction of platelets with artificial surfaces, leads to platelet aggregation and consumption. The aggregated platelets may form thrombi and thromboemboli. Polypeptides of the invention may be administered to these surgical patients to prevent the formation of thrombi and thromboemboli.

Extracorporeal circulation is routinely used for cardiovascular surgery in order to oxygenate blood. Platelets adhere to surfaces of the extracorporeal circuit. Adhesion is dependent on the interaction between GPIIb/IIIa on the platelet membranes and fibrinogen adsorbed to the surface of the circuit. (Gluszko et al., Amer. J. Physiol., 1987, 252 :H, pp 615-621). Platelets released from artificial surfaces show impaired hemostatic function. Polypeptides of the invention may be administered to prevent adhesion.

Other applications of these polypeptides include prevention of platelet thrombosis, thromboembolism and reocclusion during and after thrombolytic therapy and prevention of platelet thrombosis, thromboembolism and reocclusion after angioplasty of coronary and other arteries and after coronary artery bypass procedures. In many clinical centers patients subjected to these procedures are already receiving antiplatelet drugs which are weaker inhibitors of platelet aggregation as compared to polypeptides of the present invention. Polypeptides of the invention may also be used to prevent myocardial infarction.

These polypeptides may be administered by any convenient means which will result in its delivery into the blood stream in substantial amount. They may be combined with thrombolytic agents such as plasminogen activators or streptokinase in order to inhibit platelet aggregation. They may also be combined with anticoagulants such as heparin, aspirin or warfarin. Intravenous administration is presently contemplated as the preferred administration route. They are soluble in water, and may therefore be effectively administered in solution.

In one exemplary application, a suitable amount of Echistatin is intravenously administered to a heart attack victim undergoing angioplasty. Administration occurs during or several minutes prior to angioplasty, and is in an amount sufficient to inhibit platelet aggregation, e.g. an amount which achieves a steady state plasma concentration of between about $0.05 - 2$ µM. Should the patient need to undergo bypass surgery, Echistatin administration may be stopped immediately and will not cause complications during surgery that would be caused by other materials such as aspirin or monoclonal antibodies (e.g. 7E3, see Gold et al.) the effects of which last hours after cessation of administration.

The present invention also includes a composition comprising recombinant single-chain tissue-type plasminogen activator or streptokinase and a polypeptide having a sequence H$_2$N-(Ch)-Cys-R-R-R-Arg-Gly-Asp-R-R-R-R-R-Cys-(Cx)-H, where each R, either the same or different, represents any amino acid. The invention also includes a method for promoting thrombolysis and preventing reocclusion in a patient which comprises administering to the patient an amount of recombinant single-chain tissue-type plasminogen activator and an amount of a peptide having a sequence H$_2$N-(Ch)-Cys-R-R-R-Arg-Gly-Asp-R-R-R-R-R-Cys-(Cx)-H, where each R, either the same or different, represents any amino acid.

Polypeptides of the invention may also be administered to modulate osteoclast cellular adhesion to bone.

Osteoclasts, large multinucleated cells up to 400 microns in diameter and with abundant acidophilic cytoplasm which function in the absorption and removal of osseous tissue, use the integrin receptor to bind bone as part of the mechanism of bone resorption. The bone slice assay (Arnett and Dempster, Endocrinology 120 : 19827, 1988) shows that Echistatin inhibits the excavation of bone slices by rat osteoclasts with IC$_{50}$ of 0.1 nM. Based on the release of [$^3$H] proline from labelled bone particles, the IC$_{50}$ of chicken osteoclast resorption was 100 nM. By comparison, the RGDS synthetic tetrapeptide inhibited rat or chicken resorption with an IC$_{50}$ of 0.1 mM. Attachment experiments with chicken osteoclasts and bone particles labelled with [$^3$H] proline confirmed that the mechanism of action of echistatin is to inhibit osteoclast binding to bone. Echistatin induces the retraction of osteoclast lamellipodia in a manner reminiscent of 1 nM calcitonin or 1 uM prostaglandin E$_2$.

## Example 21

Osteoclasts were isolated from the long bones of 1-3 day old rats as described by Chambers et al., J. Cell Sci. 66 : 383-399 (1984). Femora, tibiae and humera were dissected clean of soft tissue, split and scrapped with scalpels into 199 media pH 7-7.2 + 10% heat inactivated fetal calf serum + 100 U/ml antibiotics (penicillin G, steptomycin, amphotericin B, Gibco, New York). After gentle pipetting 60x with a wide bore pipette, the cell suspension was passed through a 110 um nylon mesh (Spectra) and aliquoted onto plastic dishes (Costar, Cambridge, MA), No. 1 coverslips (Corning, New York), glass cuvettes (Herminus) or bone slices (Arnett and Dempster, Endocrinology 120 : 602-608 (1987)). All experiments with rat osteoclast cultures were conducted within the first 24 hr after isolation.

Osteoclasts from the long bones of egg laying hens were isolated by the methods of Zambonin Zallone et al. (1989) and Blair et al., J. Cell Biol. 102 : 1164-1172 (1986). Medullary bone was harvested with scalpels from split femora and tibiae of laying hens (Dekalb XL) that were on a Ca deficient diet (5070C- 9, Purina Mills Inc., St. Louis) for 3-6 wks. After washing in PBS 4°C, the suspension was pressed through a 110 um nylon mesh and then osmotically shocked in 0.2% NaCl for 3 min 37°C to lyse red blood cells. The cells were collected at 350xg (5 min, 4°C), and osteoclasts were sedimented through 70% serum for 90 min at 4°C in 50 ml tubes (Fisher). The bottom 5 ml was then layered over a 3 step (1.073, 1.099, 1.143 g/cm³) Nycodenz gradient (Accurate, Ct) and spun at 350xg, 20 min, 4°C. Cells from below the first band (monocytes) to above the pellet were pooled and resuspended into alpha-MEM pH 7- 7.2 + 10% serum + antibiotics (Gibco) + 5 ug/ml cytosine-1-B- D- arabinofuranoside (Sigma), 4°C. Cells excluding trypan blue (Gibco) were immediately counted on a hemocytometer (Fisher) and aliquoted at 1-2x 10$^6$ total cells (not counting red blood cells)/ well of 48 well plates (Costar, no. 1 coverslips or cuvettes (Fisher). Osteoclasts typically comprised 10- 50% of the total cell population although exceptional yields were occasionally observed.

## Resorption Assays

Resorption of rat and some chicken osteoclasts was measured by using bone slices (4.4 x 4.4 x 0.2 mm) from the diaphysis of bovine femur (Isomet, Buehler Ltd., Lake Bluff, IL) which were cut with a low speed diamond saw as described by Arnett and Dempster, 1986. Slices stored in 70% ethanol, 4°C, were rehydrated into 0.1 ml complete 199 media for rat cells or complete alpha- MEM media for chicken cells in a 96 well plate (Costar). After 15-18 hr incubation in moist $CO_2$- air 37°C with rat osteoclasts and RGD containing peptides (Merck), bone slices were devitalized, fixed, dehydrated and stained with 1% toluidine blue as described in Arnett and Dempster (1986). Resorption pits were quantitated by reflected light microscopy with crossed polarizers and rotatable lambda/4 plate (Sato and Grasser, J. Bone Min. Res. in press).

Resorption by chicken osteoclasts was followed by measuring release of $^3$H proline from labelled bone particles as described in Blair et al. (1986). Chicken cells in 48 well plates (Costar) were washed 3x in complete alpha- MEM media and then incubated with 100 ug of 20- 53 um particles of crushed rat bone labelled with $^3$H proline for 1-3 days. Bones labelled with $^3$H proline was made by injecting $^3$H proline (1 mCi/rat/day) into 21 day rats for 14 days ; then, bones were dissected from rats, dehydrated, crushed and seived as described in Blair et al. (1986).

Examination of resorption as a function of time (Graphs 1a and 1b) showed maximum osteoclastic activity of chicken cells between day 5-6 in culture. Therefore, for 1 day resorption experiments, unlabelled bone particles (50 ug, <20 um fraction) were first added to chicken cells at day 3 followed by labelled bone (100 ug, 20- 53 um) plus test compounds (Merck) at day 5. Alternatively, labelled bone (100 ug) plus compounds were added at day 3 and the media counted at day 6 with a LKB liquid scintillation counter.

Graph 1b

Days in Culture

Graph 1a

Days in Culture

### Attachment Assays

Attachment of $^3$H proline labelled bone particles (20- 53 um) was followed with time by assaying free and bound pools. Chicken osteoclasts in 48 well plates were given 100 ug of labelled bone particles. Free was defined as particles liberated by 3x swirling of cultures with media. Bound was defined as particles liberated after 6 hr incubation and 2x swirling in 1N NaOH. All cells were completely disrupted by the latter treatment. Samples were then ashed in a Packard 306 oxidizer (Camberra Inst., NJ) and counted (LKB).

### Adenylate Cyclase Assays

Chicken osteoclasts were incubated with 1 uCi [3H] adenine (NEN Research Products, Boston, MA) for 2 hr at 37°C, washed and incubated with 1 mM isobutyl methylxanthine (Sigma) in the absence or presence of test compound for 10 min. [3H] cAMP was column separated from other nucleotides and counted as described by Salomon et al., Anal. Biochem. 58 : 541 (1974).

### Reduced Echistatin

Echistatin (2 mg) dissolved in 0.4 ml of Gn buffer (6M guanidine HCl, 1 mM EDTA, 50 mM Tris pH 8.2) with 30 mg dithiothreitol was flushed and sealed with $N_2$ and incubated at 40°C for 90 min. Then 85 mg iodoacetamide was added to the mixture and incubated at 40°C for 40 min under $N_2$. The reaction mixture was chromatographed by C-18 reversed phase HPLC (Waters), fractions were dried and analyzed on a Protein Sequencer (model 470A, Applied Biosystems, see Gan et al. (1988)).

RGD containing peptides were observed to inhibit bone resorption (Graphs 2a and 2b). Chicken osteoclasts with 20- 53 um bone particles labelled with [3H] proline or rat osteoclasts on bone slices from the diaphysis of bovine femur were incubated with different concentrations of RGDS (●), echistatin (□) or prostaglandin $E_2$ (Δ). Chicken (2a) and rat (2b) osteoclasts were more sensitive to echistatin ($IC_{50}$= 0.1 uM chicken, 0.1 nM rat) than to the RGDS tetrapeptide ($IC_{50}$= 10-100 uM chicken, 100 uM rat). The resorption activity of rat osteoclasts was $10^3$x more sensitive to echistatin compared to chicken osteoclasts in 1 day resorption assays ; however in 2 of 3 data sets not shown, echistatin added to chicken osteoclasts for 3 days had insignificant effects on resorption up to 1 uM, suggesting that echistatin degrades with time in culture. By comparison, prostaglandin $E_2$ inhibited resorption of chicken osteoclasts with $IC_{50}$= 1 uM, see Chambers et al., Endocrinology 116 : 234-239 (1985), Arnett and Dempster (1987).

GRAPH 2a
Chicken

□ Echi
● RGDS
△ PGE2

GRAPH 2b
Rat

To explore the importance of the RGD sequence in synthetic echistatin in inhibiting bone resorption, osteoclasts were incubated with Ala[24] echistatin (graph 3a), in which the uncharged alanine was substituted for arginine. Substitution for arginine in the RGD sequence of echistatin resulted in insignificant effects (P>0.07, students t test) on the resorption activity of chicken (o) or rat (●) osteoclasts for the range 1pM- 10 uM. These data suggest that Arg in the RGD sequence of synthetic echistatin is critical in inhibiting bone resorption.

GRAPH 3a

Ala$^{24}$

○ Chicken
● Rat

GRAPH 3b

Rechi

To understand the structural contribution of the 4 disulfide bonds in synthetic echistatin in affecting resorption, osteoclasts were incubated with reduced echistatin (Graph 3b). All 8 cysteines in synthetic echistatin were confirmed to be carboxamidomethylated by sequence analysis while the rest of the sequence remained intact. Reduced echistatin had little inhibitory effect suggesting that the tertiary structure is also important in inhibiting resorption. An additional study showed that echistatin added to chicken osteoclasts for 3 days was indistinguishable from the dose response curves of ala$^{24}$ or reduced echistatin, suggesting that echistatin degrades over time in culture.

Attachment experiments showed that synthetic echistatin prevents osteoclasts from binding to bone (Graph 4). Free end bound particles of bone labelled with [³H] proline were assayed as a function of incubation time with chicken osteoclasts after 5-6 days culture at 37°C. Synthetic echistatin (□100 nM) significantly inhibited the attachment of bone particules to osteoclasts up to 150 min. Ala²⁴ echistatin (o) (10 nM) had no significant effect on the kinetics of bone attachment to osteoclasts. At this echistatin concentration, resorption was inhibited by 40% with little effect on osteoclast morphology. These data show that the RGD sequence is necessary for osteoclast attachment to bone and suggest that inhibition of resorption can be attributed to prolonging the kinetics of binding to bone.

GRAPH 4

Immunofluorescence with antibodies to echistatin showed that more synthetic echistatin binds to rat osteoclasts than to nearby mononuclear cells or to chicken osteoclasts. Parallel exposures of 1 day rat and chicken osteoclasts incubated wtih synthetic echistatin (7 nM) showed greater staining of rat over chicken osteoclasts. Echistatin fluorescence was observed all over rat osteoclasts but was primarily localized to the edges of the lamellipodia and in punctate regions at the cell/glass interface in chicken osteoclasts. These structures were reminiscent of podosomes stained with antibodies to the vitronectin receptor in both rat and chicken osteoclasts (Zambonin Zallone et al. (1989)). At higher concentration of up to 10 uM echistatin, the staining of chicken osteoclasts approached that of rat osteoclasts at 7 nM. These data confirm that rat osteoclasts have higher affinity for echistatin than do mononuclear cells or chicken osteoclasts.

RGD containing peptides were observed to induce the retraction of osteoclast lamellipodia. Rat or chicken osteoclasts were incubated with different concentrations of RGDS, echistatin and Ala²⁴ echistatin. All of these peptides induced morphological changes but rat osteoclasts 1 day after isolation on glass retracted lamellipodia at 10⁵x lower concentrations of echistatin (4.3 nM) compared to RGDS peptide. At this concentration no effect on the lamellipodia was observed for mononuclear cells in the vicinity. Similar responses were observed for ala²⁴ echistatin but at higher (34 nM) concentrations. The lamellipodia of rat osteoclasts did not retract in 10 uM RGDS, 43 pM echistatin or 3.4 nM Ala²⁴ echistatin as followed for up to 2 hr. At these concentrations, extension/retraction and ruffling motility of the lamellipodia increased. Chicken osteoclasts observed after 1 day of isolation retracted lamellipodia in response to similar concentrations of RGDS peptide (0.1 mM) but required 10³x higher concentrations of echistatin (6 uM) compared to rat osteoclasts.

In order to inhibit osteoclast binding to bone substrate, a polypeptide of the invention is administered to the patient by one of several methods, including intravenous, subcutaneous, oral, rectal, topical, parenteral, ocular, nasal, buccal and the like administration. Dosage forms include tablets, troches, dispersions, suspensions, solutions, capsules, creams, ointments, aerosols and the like.

The amount of polypeptide administered is that which inhibits osteoclast cellular adhesion to bone, e.g. an amount which achieves a concentration of about 0.1 nM to 10nM, preferably about 1 nM in bone.

Suitable pharmaceutical compositions for inhibiting osteoclast cellular adhesion to bone comprise a polypeptide of the invention as the active ingredient or a pharmaceutically acceptable salt thereof, and a pharmaceutically acceptable carrier. The term "pharmaceutically acceptable salts" refers to salts prepared from pharmaceutically acceptable non-toxic acids or bases including inorganic and organic acids and bases. The compositions include compositions suitable for oral, rectal, opthalmic, pulmonary, nasal, dermal, topical or parenteral (including subcutaneous, intramuscular and intravenous) administration, although the most suitable route in any given case will depend on the nature and severity of the conditions being treated and on the nature of the active ingredient. They may be conveniently presented in unit dosage form and prepared by any of the methods well-known in the art of pharmacy.

In practical use, a polypeptide of the invention can be combined as the active ingredient in intimate admixture with a pharmaceutical carrier according to conventional pharmaceutical compounding techniques. The carrier may take a wide variety of forms depending on the form of preparation desired for adminisration, e.g., oral or parenteral (including intravenous). In preparing the compositions for oral dosage form, any of the usual pharmaceutical media may be employed, such as, for example, water glycols, oils, alcohols, flavoring agents, preservatives, coloring agents and the like in the case of oral liquid preparations, such as, for example, suspensions, elixirs and solutions ; or carriers such as starches, sugars, microcrystalline cellulose, diluents, granulating agents, lubricants, binders, disintegrating agents and the like in the case of oral solid preparations such as, for example, powders, capsules and tablets. Because of their ease of administration, tablets and capsules represent the most advantageous oral dosage unit form, in which case solid pharmaceutical carriers are obviously employed. If desired, tablets may be sugar coated or enteric coated by standard techniques.

In addition to the common dosage forms set out above, the polypeptide may also be administered by controlled release means and/or delivery devices.

Pharmaceutical compositions of the present invention suitable for oral administration may be presented as discrete units such as capsules, cachets or tablets each containing a predetermined amount of the active ingredient, as a powder or granules or as a solution or a suspension in an aqueous liquid, a non-aqueous liquid, an oil-in-water emulsion or a water-in-oil liquid emulsion. Such compositions may be prepared by any of the methods of pharmacy but all methods include the step of bringing into association the active ingredient with the carrier which constitutes one or more necessary ingredients. In general, the compositions are prepared by uniformly and intimately admixing the active ingredient with liquid carriers or finely divided solid carriers or both, and then, if necessary, shaping the product into the desired presentation. For example, a tablet may be prepared by compression or molding, optionally with one or more accessory ingredients. Compressed tablets may be prepared by compressing in a suitable machine, the active ingredient in a free-flowing form such as powder or granules, optionally mixed with a binder, lubricant, inert diluent, surface active or dispersing agent. Molded tablets may be made by molding in a suitable machine, a mixture of the powdered compound moisted with an inert liquid diluent.

A preferred method for inhibiting osteoclast cellular adhesion to bone comprises administering to a patient a composition comprising echistatin and a pharmaceutically acceptable carrier.

## EXAMPLE 22

A composition for inhibiting osteoclast cellular adhesion to bone is prepared for intravenous administration to achieve a 1nM concentration of active ingredient in bone. The composition is an isotonic solution of echistatin in sterile saline, wherein the concentration of echistatin is 1 μM.

## Claims

**1.** The use of a polypeptide having the general formula

$$X-Cys-R-R-R-Arg-Gly-Asp-R-R-R-R-R-Cys-Y$$

wherein X is H or at least one amino acid ; Y is OH or at least one amino acid ; and each R, either same or different, is any amino acid, or pharmaceutically acceptable salts of the polypeptide, for the preparation of a composition useful for inhibiting osteoclast cellular adhesion to bone.

**2.** The use of a polypeptide :

```
Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Asp-Met-Asp-Asp-Tyr-Cys-Asn-Gly-Lys-Thr-
Cys-Asp-Cys-Pro-Arg-Asn-Pro-His-Lys-Gly-Pro-Ala-
Thr
```

or pharmaceutically acceptable salts of the polypeptide, for the preparation of a composition useful for inhibiting osteoclast cellular adhesion to bone.